# EUROPEAN PATENT SPECIFICATION

(11) **EP 4 340 756 B1**
(45) Date of publication and mention of the grant of the patent: **05.11.2025**
(21) Application number: 21729428.9
(22) Date of filing: 21.05.2021
(51) Int. Cl.: A61B 17/34, A61B 17/00

(54) **ACCESS KIT FOR CREATING A TRANSJUGULAR INTRAHEPATIC PORTOSYSTEMIC SHUNT (TIPS)**
ZUGANGSKIT ZUR ERZEUGUNG EINES TRANSJUGULÄREN INTRAHEPATISCHEN PORTOSYSTEMISCHEN SHUNTS (TIPS)
KIT D'ACCÈS POUR CRÉER UN SHUNT PORTOSYSTÉMIQUE INTRAHÉPATIQUE PAR VOIE TRANSJUGULAIRE (TIPS)

(43) Date of publication of application: 27.03.2024
(73) Proprietor: Angiomed GmbH & Co. Medizintechnik KG, 76227 Karlsruhe (DE)
(72) Inventor: FORREITER, Roman, 76227 Karlsruhe (DE)
(74) Representative: Hoffmann Eitle
(86) International application number: PCT/EP2021/063619
(87) International publication number: WO 2022/242874

(56) References cited:
- WO-A1-2008/121888
- WO-A1-2010/115134
- US-A1- 2016 361 088
- US-A1- 2017 049 511
- US-A1- 2019 184 136

## Description

### TECHNICAL FIELD

The present invention relates to an access kit for creating a TIPS shunt.

### TECHNICAL BACKGROUND

In patients suffering from liver conditions such as liver cirrhosis, a condition known as portal hypertension occurs rather frequently. This condition often leads to intestinal bleeding, potentially life-threatening oesophageal bleeding and the build-up of fluid within the abdomen.

In order to treat this condition, an artificial channel is established through the liver that establishes communication between the inflow portal vein and the outflow hepatic vein. Inside this channel, a stent graft that is typically partially covered with ePTFE is placed that keeps the channel open and that allows blood to bypass the diseased liver by flowing from the portal vein directly to the hepatic vein without having to pass through the liver.

In order to place such a TIPS shunt, an artificial channel needs to be created through the liver. In order to do so, a TIPS access kit which has the form of an access needle is advanced through the patient's vasculature from an incision site that is typically at the patient's neck and then to a location within the hepatic vein. At that point, a surgeon attempts to puncture the liver using the access needle so as to reach the portal vein. However, frequently, several attempts need to be made to successfully puncture the portal vein which each require a withdrawal of the access needle and a manual bending thereof. It is clear that the time taken for withdrawing the needle and for reinserting it complicates and prolongs the procedure. Further, there is also the risk of the surgeon puncturing themselves when bending the withdrawn access needle, which comes with the risk of exposing the surgeon to the blood of the patient, which may contain pathogens such as HCV viruses. Accordingly, it is clear that an unnecessary withdrawal of the needle should be avoided. US 2016/361088 A1 discloses a catheter for body lumen access being able to passively transition from a linear shape into a nonlinear shape or vice versa. Similar devices are disclosed in US 2019/184136 A1, WO 2010/115134 A1, WO 2008/121888 A1, and US 2017/049511 A1.

### SUMMARY OF THE INVENTION

The present invention aims at alleviating or even overcoming at least some of the problems mentioned above.

The invention is defined by the access kit as defined in claim 1. Embodiments are set out in the dependent claims.

Accordingly, an access kit for creating a TIPS shunt comprises a needle for puncturing diseased liver tissue. This needle is that component of the access kit that actually punctures the liver tissue. The needle should be designed so that it can be advanced through the patient's vasculature. Accordingly, it needs to be sufficiently bendable and low-profile. On the other hand, the needle needs to be sufficiently rigid to be able to pierce through the diseased and thus rather stiff liver tissue.

The needle comprises a first and a second lumen that extend longitudinally through the needle. At a first longitudinal end of the needle, the needle is arranged for puncturing tissue (so that this end is pointed) whilst the second longitudinal end is arranged for manipulation by a user. The user can grip this second longitudinal end for advancing and retracting the needle as well as rotating the needle about its longitudinal axis.

The kit furthermore comprises one or more stylets which are suitable for being advanced through the first lumen of the needle. By this advancement of the stylet, the curvature of the needle is changed. It is both envisaged that the curvature of the needle increases so that the needle becomes more bent or that the curvature of the needle decreases so that the needle becomes straighter. It is to be understood that the stylet does not have to extend through the entirety of the needle. Rather, it suffices if it only extends through parts of the needle. For example, the stylet does not need to reach the first longitudinal end of the needle.

In that way, when the needle has been advanced to the hepatic vein, and if it is found by the surgeon that the portal vein has not been punctured, it is possible for the surgeon to adjust the angle of bend of the needle by means of introducing a stylet. In that way, it becomes possible to change the angle of the needle in situ and to not have to withdraw it for changing its curvature. Furthermore, the access kit can be arranged so that, as the stylet is advanced through the needle, the change in curvature becomes more pronounced (so that, in the case of a needle that is, in the unconstrained state, bent, it becomes straighter and straighter as the stylet advances or that becomes more and more bent as the needle advances). That is, by advancing the stylet inside the needle, a gradual adjustment of the angle of curvature of the needle is possible. Further, thanks to the reduced time taken for changing the curvature of the needle, patient trauma and discomfort are reduced.

In embodiments, the needle is, in an unconstrained state, bent. By the unconstrained state, a state is being referred to in which no force and/or torque are applied to the needle. In that context, the curvature of the needle is reduced or increases as one of the one or more stylets is advanced through the first lumen. Accordingly, the needle becomes either straighter or more bent as the stylet is advanced through the first lumen. In embodiments, the bent state of the needle defines the maximum angle of deflection achievable with such a design if the stylets are straight.

In the context of the previous embodiment, it is possible to have more than one stylet. Each of those stylets should have the capability of adjusting the curvature of the needle. The stylets should be different from each other in how they adjust the curvature of the needle - for example, they could differ in their stiffness (for example by using materials having different Young's moduli and/or different thicknesses for the different stylets), with stiffer stylets being able to impart a more significant change in the curvature than softer stylets.

By having the needle bent in an unconstrained state, the needle is, already in the unconstrained state, suitable for creating a channel for a TIPS shunt, given that most TIPS access kits have a pre-bent needle. Accordingly, such an access kit is easier to use since one does not need to introduce a stylet for bending the needle. Accordingly, the access kit is suitable for use without using a stylet.

In that context, it is according to embodiments that in the unconstrained state, the needle has an angle of deflection in the range of from 20° to 50°, and, in more specific embodiments, in the range of from 35° to 50°. Such an angle of deflection is particularly useful for creating channels for TIPS shunts.

Alternatively, it is according to embodiments that the needle is straight in an unconstrained state and that the curvature of the needle increases as one of the one or more stylets is advanced through the first lumen. Such a design is more space efficient to package since a pre-bent needle consumes more packaging space.

It is according to embodiments if the needle changes its angle of deflection by at least 2°, optionally at least 3°, and in further embodiments at least 5° as one or, in embodiments, all of the one or more stylets are fully inserted into the first lumen. In that context, the angle of deflection is the angle between the needle at the first longitudinal end and needle at the second longitudinal end. Having such a change in angle of deflection leads to an overall change in angle that is particularly useful for surgeons, since this is a typical range within which one needs to manipulate the curvature of the needle.

It is according to embodiments that as one, in embodiments every single one, of the one or more stylets is advanced through the first lumen, the curvature of the needle changes in the region adjacent to the first longitudinal end. That is, the curvature changes in that region which is also used for puncturing the liver. Accordingly, the shape is modified in that region where it needs to be modified the most for puncturing liver tissue. Such an access kit is particularly useful in surgery. The region adjacent to the first longitudinal end is, in embodiments, defined to be that region within 20% of the length of the needle from the first longitudinal end. In further embodiments, the region adjacent to the first longitudinal end is a region within the last 25 mm, in further embodiments the last 5 to 15 mm from the first longitudinal end, as measured along the length of the needle, which is a region that is of particular importance for surgeons.

In embodiments, the first and second lumens end at the first longitudinal end of the needle. Accordingly, since they both end at the first longitudinal end of the needle, they can both be used for aspirating blood which allows for checking whether the portal vein has been punctured. Additionally, they can both be used for advancing a guidewire. Both of these capabilities are particularly important when creating TIPS shunts since a guidewire is almost always used. It is particularly relevant if the second lumen is arranged so that it ends at the first longitudinal end of the needle since typically, the first lumen will be occupied by the stylet so that it will be difficult, if not impossible, to aspirate blood and/or to advance a guidewire through it. It is also according to embodiments if the first lumen ends at the first longitudinal end of the needle. This is because by having it end at that portion, the stylet can be advanced, if it is sufficiently long, right up to the first longitudinal end, which allows for shaping the needle also at the portion that is used for puncturing.

It is also according to embodiments that the first and/or second lumen end at the second longitudinal end of the needle, which is that end that is meant for being manipulated by the user. In that way, it is easier for the user to manipulate both the stylet and/or the guidewire, since they will both extend out of that end that would be used for manipulating the needle anyway. It is also possible to easily aspirate blood since all of the manipulation occurs at that end of the needle that is meant for being manipulated by the surgeon anyway.

It is according to embodiments that the access kit further comprises a handle that is arranged at the second longitudinal end of the needle. The handle allows for manipulating the needle and thus enhances the user-friendliness for the surgeon. The handle is also arranged so that using that handle, one or more stylets can be introduced into the first lumen. For example, a funnel-shape can be arranged that would guide the stylets into the first lumen. Again, this improves user friendliness.

It is also according to embodiments if the access kit comprises a guidewire that can be advanced through the second lumen. Such a guidewire helps significantly in correctly placing the TIPS stent grafts.

In embodiments, the handle is arranged to also allow the introduction of the guidewire into the second lumen. In further embodiments, the handle could comprise a funnel-shape to ease the introduction of the guidewire into the second lumen. These features make the system more user-friendly for a surgeon.

It is additionally according to embodiments if the access kit comprises an introducer sheath that is arranged so as to surround the needle. Such an introducer sheath is helpful in access kits and serves to protect the blood vessels from combing into contact with the needle. It is also according to embodiments that the needle shaft, which is that component of the needle that is meant for puncturing liver tissue, is made of nitinol. Such a comparatively flexible material has been shown to be suitable for puncturing liver tissue whilst also being flexible enough to allow for being deformed by a stylet. The stylets could be made of or comprise a steel wire. Alternatively, the stylets could be made of other metals as long as they are stiff enough. It is also possible to use sufficiently stiff plastic materials. The needle shaft could also be made of other materials apart from nitinol. Some examples of materials to be used for the needle shaft are plastic materials that have been reinforced with carbon fibers.

It is also according to embodiments if one or more, optionally all, of the one or more stylets have a length that is at least 80%, in further embodiments at least 90% of the length of the needle. In that way, the stylets can be advanced through essentially the entirety of the needle. This allows for shaping essentially the entirety of the needle. The one or more stylets should, in embodiments, be shorter than the needle to prevent the stylet from sticking out at the distal end of the needle when fully inserted.

It is also envisaged that the needle can comprise two inner tubes that are arranged adjacent to each other inside a larger outer tube, with the interstices between the two inner tubes and the outer tube filled with a suitable filling material. In that configuration, the two inner tubes and the outer tube could be made of any of the materials mentioned before as being suitable for use for the needle shaft. In embodiments, the two inner tubes and the other tube would be made of nitinol and/or stainless steel. The filling material could be a suitable plastic material, which would, in some embodiments, be injection moulded into the interstices. In such a configuration, one of the two inner tubes could be used for advancing a guidewire whilst the respective other inner tube would be used for advancing a stylet. Such a needle would be comparatively easy to manufacture. By having the filling material fill the interstices, an intrusion of bodily fluids into the needle is prevented. Further, the inner and outer tubes are stabilized in their respective position.

In further embodiments, the needle comprises an end section member arranged at a distalmost end of the needle. The end section member is, in embodiments, made of a harder material than a material of the needle shaft. With such a configuration, it becomes possible to have a harder material at the tip of the needle, which is thus more suitable for penetrating tissue. Such a material can, in embodiments, be made of or comprise a ceramic material. Whilst ceramic materials are often very brittle and cannot be easily bent, which renders them difficult or impossible to use for the needle shaft, they are also often very hard, which makes them promising materials for penetrating through tissue. Accordingly, they can, according to the embodiment, be combined with more flexible materials to be used in the needle shaft. Thus, a hard and thus highly penetrative needle tip can be combined with a flexible needle shaft.

### BRIEF DESCRIPTION OF THE DRAWINGS

Figure 1 shows a TIPS access kit according to a first embodiment of the invention in different states.
Figures 2 and 3 show a distal end of the access kit shown in Figure 1.
Figure 4 shows the TIPS access kit in different states.
Figure 5 shows the TIPS access kit according to a second embodiment of the invention.
Figure 6 shows the TIPS access kit according to a third embodiment of the invention.
Figure 7 shows the assembled TIPS access kit of Figures 2 and 3.
Figure 8 shows steps in the usage of a TIPS access kit according to the described embodiments.

### DETAILED DESCRIPTION OF THE DRAWINGS

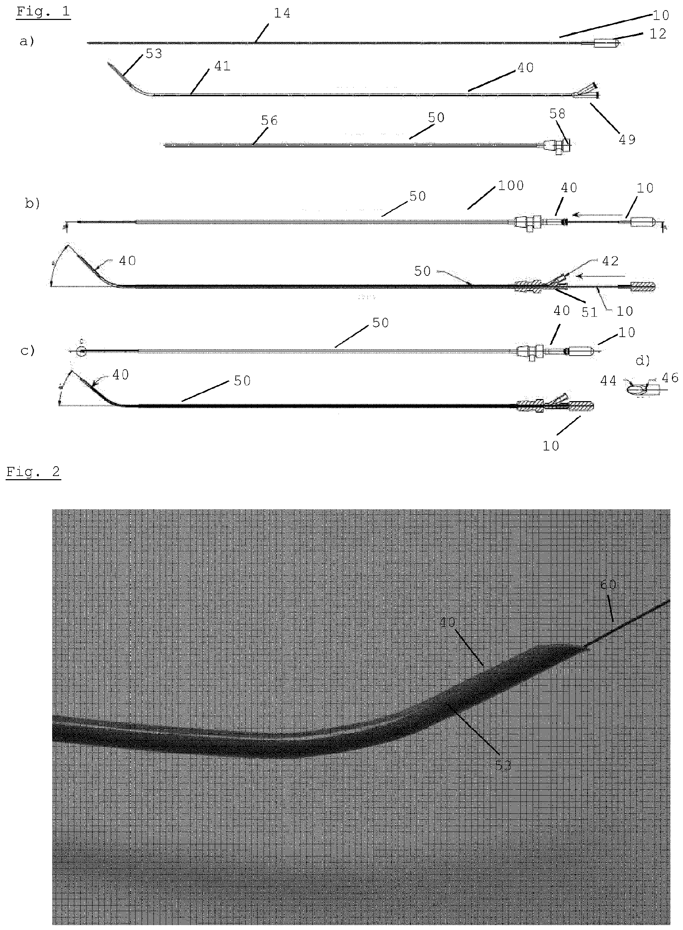

Figure 1a) shows a TIPS access kit according to a first embodiment of the invention.

A needle 40 is shown that comprises a shaft 41 that is attached to a handle 49 which is arranged at a proximal end (second longitudinal end) of the needle 40. A distal end 53 of the shaft 41 of the needle 40, which is also referred to as the first longitudinal end of the needle 40, is bent. There is furthermore provided one stylet 10 having a handle 12 and a metal wire 14. The metal wire 14 is arranged so that it can be advanced through the first lumen of the needle 40 (to be discussed below) by means of being introduced into the first lumen via the handle 49. There is furthermore provided an introducer sheath 50 having an introducer sheath handle 58 and an introducer sheath shaft 56. The introducer sheath 50 can be slid over the shaft 41 of the needle 40 by means of the shaft 41 being inserted into a corresponding opening of the handle 58 of the introducer sheath 50.

Figures 1b) and c) show a state in which the stylet 10, the needle 40, and the introducer sheath 50 shown in Figure 1a) have been slid into each other so as to create a configuration that could be used in a TIPS procedure. That is, the introducer sheath 50 has been slid over the needle 40 whilst the stylet 10 has been partially introduced into the first lumen of the needle 40.

As can be seen from Figure 1b), where the top drawing shows a view in which a person looks down straight onto the needle so that it appears straight, the introducer sheath 50 has been fully advanced onto the needle 40 so that the introducer sheath handle 58 abuts against handle 49 of the needle 40.

Figure 1c) shows the state in which the stylet 10 has been fully inserted and in which the stylet handle 12 abuts against the needle handle 49. As can be seen from the distal end 53 of the needle 40, the angle of curvature of the distal end 53 has been reduced, when compared with Figure 1b). Put differently, the needle 40 has been brought into a straighter state. Accordingly, if a surgeon finds out that the needle 40 will not puncture the portal vein in the state shown in Figure 1b), they can fully advance the stylet 10 into the needle 40 to thereby make the needle 40 somewhat straighter, as indicated by Figure 1c). In this straighter state of the needle 40, a puncture of the portal vein may be possible without having to withdraw the needle 40 and manually bending it.

Figure 1d) shows the distal-most end of the needle 40. As can be seen, there is an opening of the first lumen 44 as well as a (smaller) opening of the second lumen 46. The first lumen 44 is used for the stylet 10 whilst the second lumen 46 is used for a guidewire and/or for the aspiration of blood from the portal vein to indicate a successful puncture of the portal vein.

Figure 2 shows a close-up of the distal end 53 of the needle. As can be seen in this close-up, the distal end 53 of the needle 40 is bent. Furthermore, a guide wire 60 has been advanced through the second lumen.

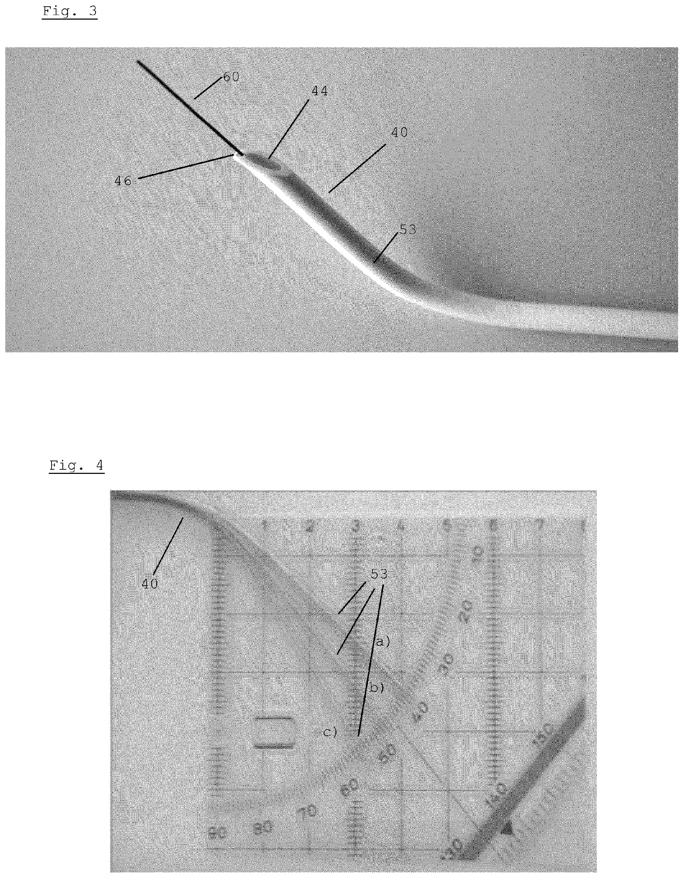

This configuration can be seen more clearly from Figure 3, where it can be seen that the guide wire 60 extends out of the second lumen 46 whilst the distal end of the first lumen 44 is empty.

Figure 4 shows the bent distal end 53 of the needle 40 in different states of insertion of one of the stylets 10. Whilst the state c) shows the greatest amount of change in the curvature of the distal end 53 of the needle, the distal end is bent less in state b) and even less so in state c).

The needle 40 transitions from the state shown in Figure 4, state c) to the state shown in b) and a) by progressive insertion of the stylet 10 (not shown). As can be seen from the goniometer that is also shown in Figure 4, the reduction in the angle of deflection is significant.

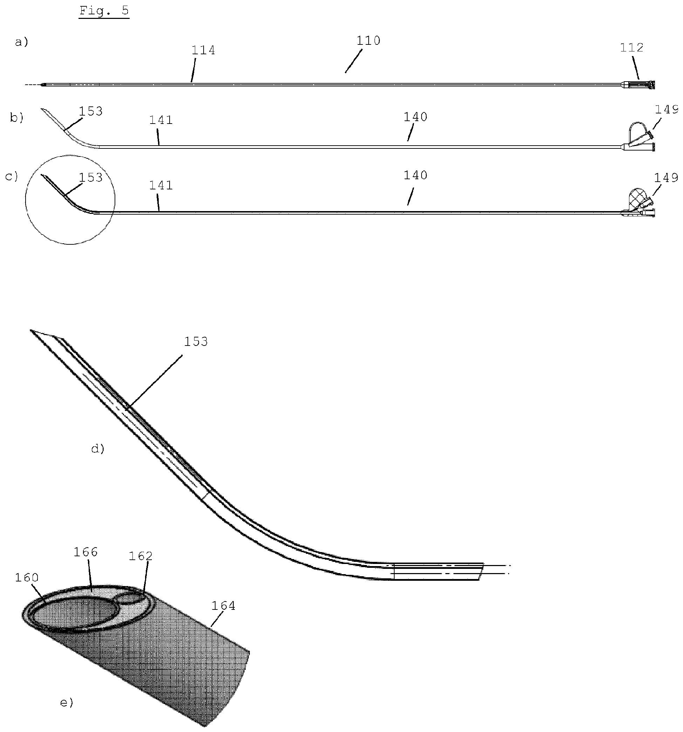

Figure 5 shows, in sub-figures a)-e), components of a TIPS access kit according to a second embodiment of the present invention. It is to be noted that, compared with the first embodiment, the same reference numerals have been used as in the first embodiment, with "100" being added.

Figure 5a) shows a stylet 110 to be used in the TIPS access kit according to the second embodiment. This stylet 110 is identical to the stylet 10 described with respect to the first embodiment.

The needle 140 that is shown in Figures 5b) and 5c) differs somewhat in its details from the needle 40 that was described with respect to the first embodiment. Again, the needle 140 comprises a shaft 141 and a handle 149. A distal end 153 of the needle 140 is bent, as can also be seen in Figure 5d).

The differences between the needle 140 of the second embodiment and the needle 40 according to the first embodiment can be seen more clearly from Figure 5e). Whilst the needle 40 of the first embodiment has a shaft 41 that was made of a monolithic body, the shaft 141 of the needle 140 according to the second embodiment is made of several components. As can be seen from Figure 5e), inside an outer tube 164, two inner tubes 160,162 are arranged adjacent to each other. Those two inner tubes 160, 162 extend along the whole length of the outer tube 164. The interstices between the two inner tubes 160, 162 and the outer tube 164 are filed with a filling material 166. In the presently envisaged embodiment, the outer tube 164 and the two inner tubes 160, 162 are made of stainless steel, whilst the filling material 166 is a plastic material.

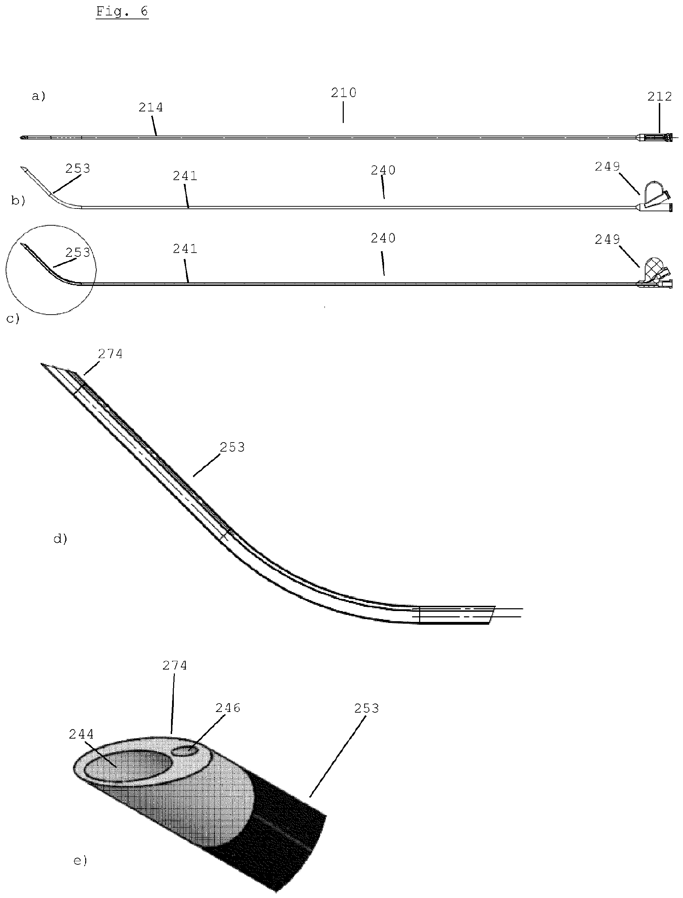

Figure 6 shows, in sub-figures a)-e), components of a TIPS access it according to a third embodiment. Again, the reference numerals are identical to those used in the first embodiment, where we have added "200" so as to be able to distinguish between them.

As can be seen from Figure 6a), a stylet 210 comprises a metal wire 214 that extends from a handle 212. This stylet 210 can be introduced into the needle 240 that is depicted in Figures 6b) and 6c). Again, a shaft 241 extends from a handle 249 of the needle 240. A distal end 253 of the needle 240 is bent. The angle of bent of the distal end 253 can be adjusted by means of introducing the stylet 210 into one of the lumens of the needle 240.

The distal end 253 of the needle 240, which is encircled in Figure 6c), is shown in an enlarged form in Figure 6b). In that figure, it can be seen that the distal end 253 comprises, at the distalmost end, a distal end section member 274 which is shown in more detail in Figure 6e). As can be seen from that sub-figure, the distal end section member 274 is arranged at the distalmost end of the distal part 253 of the needle 240. The distal end section member 274 can be made of, for example, ceramics or any other kind of material that has a higher hardness than a metallic material but which would not be suitable for use as the remainder of the shaft of the needle shaft 241 due to its low flexibility. As can also be seen from that Figure, the first lumen 244 and the second lumen 246 extend through the distal end section member 274.

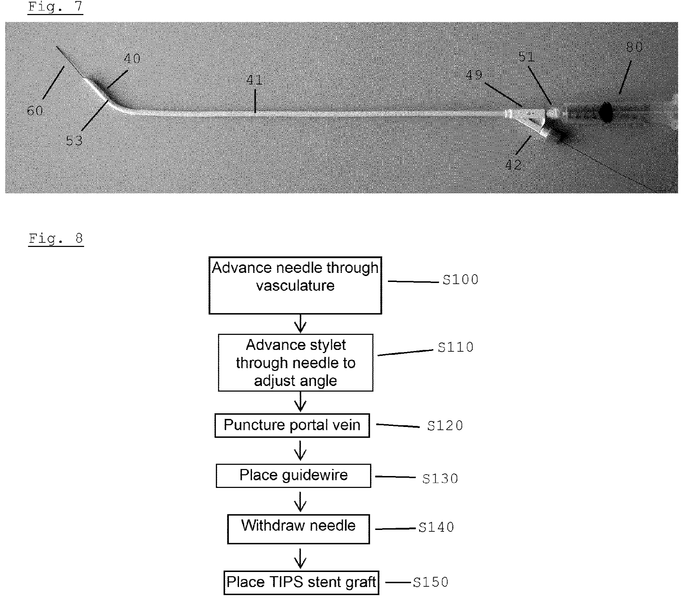

Figure 7 shows a view of a prototype of the entire system where components are depicted in Figures 2-4. It is to be noted that also the components depicted in figures 5 and 6 could be arranged in the manner shown in Figure 7.

As can be seen, a needle 40 having a shaft 41 is provided where that shaft 41 extends from a needle handle 49. The needle handle 49 has two inlets, namely a first inlet 51 that is connected to the first lumen 44 and a second inlet 42 that is connected to the second lumen 46 and through which the guide wire 60 extends. The guidewire 60 extends out of the bent distal end 53 of the needle, as can be seen from Figure 5. At the first inlet 51 of handle 49, a syringe 80 is used. The syringe 80 is connected, using the first inlet 51, to the first lumen 44, which can therefore also be used for aspirating blood.

Figure 8 shows steps that can be used in using the system according to the embodiments described previously.

First of all, a puncture is created in a patient's skin that allows for accessing their vasculature.

Through that puncture, the assembled access kit, which comprises a needle 40 that is inserted into an introducer sheath 15, is advanced so that the tip of the needle 40 reaches the hepatic vein (step S100).

Then, optionally, a puncture attempt is made at the portal vein. If that puncture attempt fails, or otherwise by default, a stylet 10 is advanced through the first lumen so as to adjust the angle of the distal end of the needle 40 (step S110).

Subsequently, a puncture attempt of the portal vein is made using the needle 40. If need be, step S110 and S120 can be repeated as often as needed until the portal vein is punctured (Step S120).

In that state, a guide wire is advanced through the second lumen 46. Once the guide wire has been placed (step S130) the needle 40 and the introducer sheath 50 are withdrawn, together with any stylets 10.

Subsequently, using a suitable TIPS Stent graft delivery device, a TIPS stent graft is placed where the delivery device is advanced over the guidewire (step S150).

Once the TIPS stent graft has been placed, the guide wire as well as the TIPS stent graft delivery system are withdrawn from the patient's vasculature, and the incision is closed so as to allow it to heal.

It is envisaged that the needle 40 will comprise geographic alignment markers and/or materials that make the needle 40 visible under fluoroscopy. For examples, fluoroscopically visible rings or circular markers could be provided, e.g., close to the tip of the needle 40 or further towards the handle of the needle 40.

## Claims

1. Access kit (100) for creating a TIPS shunt, comprising:
- a needle (40) for puncturing diseased liver tissue and being designed for being advanced through a patient's vasculature, the needle (40) comprising a first (44) and a second (46) lumen extending longitudinally through the needle (40), the needle having a first longitudinal end (45) arranged for puncturing tissue and a second longitudinal end (47) arranged for manipulation by a user,
- one or more stylets (10), the stylets (10) being suitable for being advanced through the first lumen (44),
wherein the needle (40) and the one or more stylets (10) are arranged so that, as one of the one or more stylets (10) is advanced through the first lumen (44), the curvature of the needle (40) changes.

2. Access kit according to claim 1, wherein the needle (40) is bent in an unconstrained state and wherein the curvature of the needle is reduced or increases as one of the one or more stylets (10) is advanced through the first lumen (44).

3. Access kit according to claim 2, wherein in the unconstrained state, the needle (40) has an angle of deflection in the range of from 20° to 50°, optionally in the range of from 35° to 50°.

4. Access kit according to claim 1, wherein the needle (40) is straight in an unconstrained state and wherein the curvature of the needle increases as one of the one or more stylets is advanced through the first lumen.

5. Access kit according to one of the preceding claims, wherein the needle (40) changes its angle of deflection by at least 2°, optionally at least 3°, further optionally at least 5°, as one, optionally all, of the one or more stylets (10) is advanced through the first lumen (44).

6. Access kit according to one of the preceding claims, the kit being arranged so that as one of the one or more stylets is advanced through the first lumen (44), the curvature of the needle changes in the region adjacent to the first longitudinal end, wherein the region adjacent to the first longitudinal end is optionally defined to be the region within 20% of the length of the needle from the first longitudinal end.

7. Access kit according to one of the preceding claims, the first (44) and/or second (46) lumen being arranged so that they end at the first longitudinal end of the needle.

8. Access kit according to one of the preceding claims, the first (44) and/or second (46) lumen being arranged so that they end at the second longitudinal end (47) of the needle.

9. Access kit according to one of the preceding claims, further comprising a handle (49) arranged at the second longitudinal end of the needle (40), the handle being arranged for allowing the introduction of one of the one or more stylets (10) into the first lumen.

10. Access kit according to one of the preceding claims, further comprising a guidewire (60) that can be advanced through the second lumen (46).

11. Access kit according to claim 10, when also comprising the features of claim 9, wherein the handle (49) is arranged for allowing the introduction of the guidewire (60) into the second lumen.

12. Access kit according to one of the preceding claims, further comprising an introducer sheath (50) arranged so as to surround the needle (40).

13. Access kit according to one of the preceding claims, the needle (40) having a shaft (41) arranged for puncturing diseased liver tissue, wherein the shaft (41) is made of nitinol.

14. Access kit according to one of the preceding claims, wherein one or more of the one or more stylets (10) has a length that is at least 90% of the length of the needle (40).

15. Access kit according to one of the preceding claims, the needle comprising an outer tube (164) and one or two inner tubes (160, 162) arranged inside the outer tube (164), the inner tubes (160, 162) defining the first and/or the second lumen (144, 146).

## Patentansprüche

1. Zugangskit (100) zur Erzeugung eines transjugulären intrahepatischen portosystemischen Shunts, umfassend:
- eine Nadel (40) zum Punktieren von erkranktem Lebergewebe, die dafür ausgelegt ist, durch das Gefäßsystem eines Patienten vorgeschoben zu werden, wobei die Nadel (40) ein erstes (44) und ein zweites (46) Lumen umfasst, die sich in Längsrichtung durch die Nadel (40) erstrecken, wobei die Nadel ein erstes Längsende (45) zum Punktieren von Gewebe und ein zweites Längsende (47) zur Manipulation durch einen Benutzer aufweist,
- ein oder mehrere Stifte (10), wobei die Stifte (10) dazu geeignet sind, durch das erste Lumen (44) vorgeschoben zu werden,
wobei die Nadel (40) und der eine oder die mehreren Stifte (10) so angeordnet sind, dass eine Veränderung der Krümmung der Nadel (40) eintritt, wenn einer der einen oder mehreren Stifte (10) durch das erste Lumen (44) vorgeschoben wird.

2. Zugangskit nach Anspruch 1, wobei die Nadel (40) in einem uneingeschränkten Zustand gebogen ist und wobei die Krümmung der Nadel verringert wird oder zunimmt, wenn eines der einen oder mehrere Stifte (10) durch das erste Lumen (44) vorgeschoben wird.

3. Zugangskit nach Anspruch 2, wobei die Nadel (40) im uneingeschränkten Zustand einen Ablenkwinkel im Bereich von 20° bis 50°, gegebenenfalls im Bereich von 35° bis 50°, aufweist.

4. Zugangskit nach Anspruch 1, wobei die Nadel (40) in einem uneingeschränkten Zustand gerade ist und wobei die Krümmung der Nadel zunimmt, wenn eines der einen oder mehrere Stifte durch das erste Lumen vorgeschoben wird.

5. Zugangskit nach einem der vorstehenden Ansprüche, wobei die Nadel (40) ihren Ablenkungswinkel um mindestens 2°, gegebenenfalls um mindestens 3°, weiter gegebenenfalls um mindestens 5° verändert, wenn eines, optional alle, der einen oder mehreren Stifte (10) durch das erste Lumen (44) vorgeschoben wird.

6. Zugangskit nach einem der vorstehenden Ansprüche, wobei das Kit so angeordnet ist, dass sich die Krümmung der Nadel in der Region neben dem ersten Längsende in der Veränderung befindet, wenn einer des einen oder der mehreren Stifte durch das erste Lumen (44) vorgeschoben wird, wobei die Region neben dem ersten Längsende gegebenenfalls als die Region innerhalb von 20 % der Länge der Nadel vom ersten Längsende definiert ist.

7. Zugangskit nach einem der vorstehenden Ansprüche, wobei das erste (44) und/oder zweite (46) Lumen so angeordnet sind, dass sie am ersten Längsende der Nadel enden.

8. Zugangskit nach einem der vorstehenden Ansprüche, wobei das erste (44) und/oder zweite (46) Lumen so angeordnet sind, dass sie am zweiten Längsende (47) der Nadel enden.

9. Zugangskit nach einem der vorstehenden Ansprüche, weiter umfassend einen Griff (49), der am zweiten Längsende der Nadel (40) angeordnet ist, wobei der Griff so angeordnet ist, dass er das Einführen eines des einen oder der mehreren Stifte (10) in das erste Lumen ermöglicht.

10. Zugangskit nach einem der vorstehenden Ansprüche, weiter umfassend einen Führungsdraht (60), der durch das zweite Lumen (46) vorgeschoben werden kann.

11. Zugangskit nach Anspruch 10, wenn es auch die Merkmale von Anspruch 9 umfasst, wobei der Griff (49) so angeordnet ist, dass er das Einführen des Führungsdrahts (60) in das zweite Lumen ermöglicht.

12. Zugangskit nach einem der vorstehenden Ansprüche, weiter umfassend eine Einführhülse (50), die so angeordnet ist, dass sie die Nadel (40) umgibt.

13. Zugangskit nach einem der vorstehenden Ansprüche, wobei die Nadel (40) eine Welle (41) aufweist, die zum Punktieren von erkranktem Lebergewebe angeordnet ist, wobei die Welle (41) aus Nitinol besteht.

14. Zugangskit nach einem der vorstehenden Ansprüche, wobei eine oder mehrere der einen oder mehreren Stifte (10) eine Länge aufweisen, die mindestens 90 % der Länge der Nadel (40) beträgt.

15. Zugangskit nach einem der vorstehenden Ansprüche, wobei die Nadel einen äußeren Schlauch (164) und einen oder zwei innere Schläuche (160, 162) umfasst, die innerhalb des äußeren Schlauchs (164) angeordnet sind, wobei die inneren Schläuche (160, 162) das erste und/oder das zweite Lumen (144, 146) definieren.

## Revendications

1. Kit d'accès (100) pour créer un shunt portosystémique intrahépatique par voie transjugulaire, comprenant :
- une aiguille (40) pour perforer un tissu hépatique malade et étant conçue pour être avancée à travers un système vasculaire d'un patient, l'aiguille (40) comprenant une première (44) et une seconde (46) lumières s'étendant longitudinalement à travers l'aiguille (40), l'aiguille présentant une première extrémité longitudinale (45) agencée pour perforer un tissu et une seconde extrémité longitudinale (47) agencée pour une manipulation par un utilisateur,
- un ou plusieurs stylets (10), les stylets (10) étant appropriés pour être avancés à travers la première lumière (44),
dans lequel l'aiguille (40) et lesdits un ou plusieurs stylets (10) sont agencés de sorte que, lorsqu'un stylet parmi lesdits un ou plusieurs stylets (10) est avancé à travers la première lumière (44), la courbure de l'aiguille (40) change.

2. Kit d'accès selon la revendication 1, dans lequel l'aiguille (40) est pliée dans un état non contraint et dans lequel la courbure de l'aiguille est réduite ou augmente lorsque l'un du un ou des plusieurs stylets (10) est avancé à travers la première lumière (44).

3. Kit d'accès selon la revendication 2, dans lequel, dans l'état non contraint, l'aiguille (40) présente un angle de déviation dans la plage de 20° à 50°, facultativement dans la plage de 35° à 50°.

4. Kit d'accès selon la revendication 1, dans lequel l'aiguille (40) est droite dans un état non contraint et dans lequel la courbure de l'aiguille augmente lorsque l'un du un ou des plusieurs stylets est avancé à travers la première lumière.

5. Kit d'accès selon l'une des revendications précédentes, dans lequel l'aiguille (40) change son angle de déviation d'au moins 2°, facultativement au moins 3°, facultativement en outre au moins 5°, lorsque l'un, facultativement la totalité, du un ou des plusieurs stylets (10) est avancé à travers la première lumière (44).

6. Kit d'accès selon l'une des revendications précédentes, le kit étant agencé de sorte que, lorsque l'un du un ou des plusieurs stylets est avancé à travers la première lumière (44), la courbure de l'aiguille change dans la région adjacente à la première extrémité longitudinale, dans lequel la région adjacente à la première extrémité longitudinale est facultativement définie comme étant la région située à moins de 20 % de la longueur de l'aiguille à partir de la première extrémité longitudinale.

7. Kit d'accès selon l'une des revendications précédentes, la première (44) et/ou la seconde (46) lumières étant agencées de sorte qu'elles se terminent à la première extrémité longitudinale de l'aiguille.

8. Kit d'accès selon l'une des revendications précédentes, la première (44) et/ou la seconde (46) lumières étant agencées de sorte qu'elles se terminent à la seconde extrémité longitudinale (47) de l'aiguille.

9. Kit d'accès selon l'une des revendications précédentes, comprenant en outre une poignée (49) agencée à la seconde extrémité longitudinale de l'aiguille (40), la poignée étant agencée pour permettre l'introduction de l'un du un ou des plusieurs stylets (10) dans la première lumière.

10. Kit d'accès selon l'une des revendications précédentes, comprenant en outre un fil-guide (60) qui peut être avancé à travers la seconde lumière (46).

11. Kit d'accès selon la revendication 10, lorsque comprenant également les caractéristiques selon la revendication 9, dans lequel la poignée (49) est agencée pour permettre l'introduction du fil-guide (60) dans la seconde lumière.

12. Kit d'accès selon l'une des revendications précédentes, comprenant en outre une gaine d'introduction (50) agencée de manière à entourer l'aiguille (40).

13. Kit d'accès selon l'une des revendications précédentes, l'aiguille (40) présentant une tige (41) agencée pour perforer un tissu hépatique malade, dans lequel la tige (41) est fabriquée en nitinol.

14. Kit d'accès selon l'une des revendications précédentes, dans lequel l'un ou plusieurs du un ou des plusieurs stylets (10) présentent une longueur qui est au moins 90 % de la longueur de l'aiguille (40).

15. Kit d'accès selon l'une des revendications précédentes, l'aiguille comprenant un tube extérieur (164) et un ou deux tubes intérieurs (160, 162) agencés à l'intérieur du tube extérieur (164), les tubes intérieurs (160, 162) définissant la première et/ou la seconde lumières (144, 146).
